# EUROPEAN PATENT SPECIFICATION

(11) **EP 2 170 084 B1**
(45) Date of publication and mention of the grant of the patent: **10.09.2014**
(21) Application number: 08718458.6
(22) Date of filing: 24.01.2008
(51) Int. Cl.: A01N 63/04, A01N 65/40, A01P 7/04

(54) **PHYTOSANITARY COMPOSITION COMPRISING AN ENTOMOPATHOGENIC FUNGUS BELONGING TO THE SPECIES B. BASSIANA AND TRITURATES OR FRAGMENTS OF DATE SEEDS, METHOD OF PREPARATION AND USE OF SAME**
PFLANZENSCHUTZZUSAMMENSETZUNG, DIE EINEN ENTOMOPATHOGENEN PILZ DER ART B. BASSIANA UND BRUCHSTÜCKE ODER FRAGMENTE VON DATTELSAMEN UMFASST, VERFAHREN ZU IHRER HERSTELLUNG UND IHRE VERWENDUNG
COMPOSITION PHYTOSANITAIRE CONTENANT UN CHAMPIGNON ENTOMOPATHOGENE APPARTENANT A L'ESPECE B. BASSIANA ET DES GRAINES DATTIER TRITUREES OU FRAGMENTEES, PROCEDE D'ELABORATION ET UTILISATION DE CELLE-CI

(30) Priority: 05.06.2007 ES 200701550
(43) Date of publication of application: 07.04.2010
(73) Proprietor: Universidad De Alicante, 03690 Alicante (ES)
(72) Inventor: ASENSIO BERBEGAL, Leticia, 03690 Alicante (ES); LÓPEZ LLORCA, Luis, Vicente, 03690 Alicante (ES); CARBONELL CARBONELL, Toñi, 03690 Alicante (ES); GÜERRI AGULLÓ, Berenice, 03690 Alicante (ES); BARRANCO VEGA, Pablo, 03690 Alicante (ES)
(74) Representative: Pons Ariño, Angel
(86) International application number: PCT/ES2008/070010
(87) International publication number: WO 2008/148920

(56) References cited:
- WO-A1-2006/121350
- WO-A2-2007/087813
- ES-A1- 2 134 174
- ES-T3- 2 084 014
- LOPEZ-LLORCA L V ET AL: "Colonization of plant waste substrates by entomopathogenic and mycoparasitic fungi a SEM study", MICRON, PERGAMON, OXFORD, GB, vol. 30, no. 4, 1 August 1999 (1999-08-01) , pages 325-333, XP007909632, ISSN: 0968-4328, DOI: 10.1016/S0968-4328(99)00031-1 [retrieved on 1999-06-23]
- SALEH M M E ET AL: "Persistence of Steinernema carpocapsae (Nematoda: Steinernematidae) and Beauveria bassiana (Deuteromycotina: Hyphomycetes) in Soil around Date Palm Trunks and their Effect on Adults of Rhynchophorus ferrugineus", EGYPTIAN JOURNAL OF BIOLOGICAL PEST CONTROL,, vol. 14, no. 1, 1 April 2004 (2004-04-01), pages 135-141, XP009120814, ISSN: 1110-1768
- BERENICE GÜERRI-AGULLÓ ET AL: "Infection of the Red Palm Weevil ( Rhynchophorus ferrugineus ) by the entomopathogenic fungus Beauveria bassiana : A SEM study", MICROSCOPY RESEARCH AND TECHNIQUE, vol. 73, no. 7, 18 December 2009 (2009-12-18), pages 714-725, XP055062236, ISSN: 1059-910X, DOI: 10.1002/jemt.20812
- LOPEZ-LLORCA L.V., CARBONELL T., SALINAS J.: 'Colonization of plant waste substrates by entomopathogenic and mycoparasitic fungi-A SEM study' MICRON. vol. 30, no. 4, August 1999, pages 325 - 333, XP007909632
- EL-SUFTY R. ET AL.: 'Biological control of red palm weevil Rhynchoporus ferrugineus (Col.: Curculionidae) by the entomopathogenic fungus Beauveria bassiana in United Arab Emirates' ACTA HORTICULTURAE no. 736, March 2007, pages 399 - 404, XP009120804
- SALEH M.M.E. ET AL.: 'Persistence of Steinernema carpocapsae (Nematoda: Steinernematidae) and Beauberia bassiana (Deuteromycotina: Hyphomycetes) in soil around data plam trunks and thei effect on adults of Rhynchophorus ferrugineus' EGYPTIAN JOURNAL OF BIOLOGICAL PEST CONTROL vol. 14, no. 1, 2004, pages 141 - 145
- GINDIN G. ET AL.: 'Evaluation of the entomopathogenic fungi Metarhizium anisopliae and Beauveria bassiana against the red palm weevil Rhynchoporus ferrugineus' PHYTOPARASITICA vol. 34, no. 4, August 2006, pages 370 - 379, XP007909631

## Description

### FIELD OF INVENTION

The present invention relates to a phytosanitary composition based on palm seed by-products and an entomopathogenic fungus (biocontrol agent) of the species *Beauveria bassiana* ((kursiv)) to reduce the populations of the insect *Rhynchophorous ferrugineus* ((kursiv)) that attack the palms.

### BACKGROUND ART

The continuing growth of human population requires finding new ways to increase food production. One way of achieving this objective is the reduction of losses in agricultural crops caused by organisms causing pests and diseases. Among the methods used to control crop damage caused by insects it is the use of agrochemicals. These products have a very important role in reducing the economic damage caused by pests on crops. However, the high toxicity of some of them, their residues and inadequate management have led to a rethinking of the tactics of biological pest control, which has led to the development of bio-insecticides that include bacteria, viruses and entomopathogenic fungi as well as natural toxins produced by microorganisms with low environmental impact.

Entomopathogenic fungi offer exciting possibilities for biological control because, in addition to the direct damage that can lead to their hosts, they can establish epizootics among in their populations. This interest for entomopathogenic fungi started ca. 100 years ago when their pathogenic capabilities were unraveled. Although they were initially used for insect pest control their performance was not satisfactory. This resulted in the development and massive use of chemical insecticides. The misuse of pesticides and its associated hazards, have raised an increasing interest in the use of fungi to control insect pests.

*Beauveria, Metarhizium, Lecanicillium* (= *Verticillium*) (Moore and Prior (1993) Biocontrol News and Information 14, 31-40), *Cordyceps* and *Paecilomyces* are among the main genera under study for their development as mycoinsecticides. Within the genus *Beauveria,* the species *Beauveria bassiana* has a wide range of hosts (Knudsen et al. (1990) Journal of Economic Entomology 83, 2225-2228) and produces mycotoxins in culture media, which when inoculated into larvae of silkworms cause swelling and stiffness.

To obtain fungal biomass for their use as bioinsecticides, it is necessary to find adequate growth media which vary depending on the species involved. In practice, the most common solid substrates used for the production of entomopathogenic fungi such as *M. anisopliae* are primary agricultural products. Besides them, some agricultural byproducts are being developed as growth media because of their low costs.

Mycotech Corporation (USA) developed a solid substrate with starch particles suitable for growth of entomopathogenic fungi. Other substrates have been developed based on various agricultural products such as wet mixtures of bran, corn flour and water and other growth media based on cotton seed cover powder, moist bran, corn meal and water. Natural products such as pine bark, coconut water (agricultural product) or almond mesocarp (Lopez-Llorca, LV and Carbonell, T. (1998). Canadian Journal of Microbiology 44, 86-90) have also been tested for the production of antagonistic fungi.

Production of species such as *M*. *anisopliae, B. bassiana, L. lecanii* and other deuteromycetes, is usually based on the fermentation of rice grains peeled and sterilized (unpolished). On this substrate yields ranging from 10 to 12% fungal mass, which is roughly 10⁹ conidia/g of rice (Alves, 1986. Editora Manole, Sao Paulo, Brazil, pp. 311-323). These substrates are usually applied directly into the soil to control insect pests underground. Other alternative substrates such as corn, soy beans and potatoes have been tested with yields lower than those obtained with rice (Mendoça et al. (1991) Biological Control of Locusts and Grasshoppers, Lomer, CJ i Prior, C. ( eds.), Wallingford, UK, pp. 239-244).

The use of *B. bassiana* strains colonised on palm-seed triturates for the control of certain pests is known (Lopez-Llorca et al. (1999) Micron 30, 325-333). Furthermore, certain strains of *B. bassiana,* derived from *Heterorhabditid* nematodes, have been used for control of red palm weevils (Saleh et al. (2004) Egyptian Journal of Biological Pest Control 14(1), 141-145).

When filamentous fungi grow in solid substrates, most mycelia develop in the free spaces between particles. Therefore a reduced availability of these (since they are readily colonised) can be a limiting factor for growth even under optimal conditions for gas exchange. It is then clear the importance of particle size in the final growth yields. When necessary the substrate can be pre-treated to increase its availability and accesibility to fungi.

### SUMMARY OF THE INVENTION

The authors of the present invention have developed a phytosanitary composition based on agricultural by-products of palmaceae and entomopathogenic fungi (biocontrol agents) to reduce populations of insects attacking palms, preferably date palm (from now onwards, *"the composition or the product formulated'*). For the development of this composition several fungus genera were tested (*Beauveria, Lecanicillium,* etc.) with the composition a number of assays were performed with several strains belonging to these genera.

In this way a composition with high virulence vs pest insects of palms plaga was obtained. The composition was efficient to control the red palm weevil (*Rhynchophorus ferrugineus*), both for larvae and adults, generating a 100% mortality in short periods of exposure. In this way, the composition is an efficient bioinsecticide which, because of its preservation, storage and production characteristics, is amenable for industrial scaling-up. The composition shows a number of advantages, which are listed bellow:
■ Allows the reduction of the numbers of pest insects plaga in palms and similar species, with no damage to the plants or to other benefitial organisms
■ Increases the survival time of the fungi in the environment due to the supply of nutrients by the substrate on which they grow (higher viability)
■ Fungi in this composition can withstand adverse environmental conditions for longer periods,
■ The use of fungi along with the substrate on which they grow in facilitates their application in the field to act against their respective target insects,
■ Allows the recycling of agricultural products (substrate on which the fungi grow and that form part of the composition of the present invention)

The present invention relates to a phytosanitary composition which includes an entomopathogenic fungus belonging to the species *B. bassiana* and palm seed by-products, wherein the entomopathogenic fungus, is selected from any of the strains with with deposit no. CBS121096 (strain 193) and CBS121097 (strain 203), deposited in the Dutch collection, "Centraalbureau voor Schimmelcultures (CBS)."

The by-product of palm seed, preferably shredded, preferably comes from *Phoenix dactylifera.*

The seed of *P. dactylifera* supplys nutrients, thus resulting in an advantageous combination with the fungus.

The phytosanitary composition in an even more preferred execution comprises powder from the crushing of the palm seeds and can protect plants from attacks by the red weevil (*Rhynchophorus ferrugineus*).

A second aspect of the invention relates to the method for obtaining a phytosanitary composition comprising:
a. Fragmenting or crushing palm seed, preferably of the species *Phoenix dactylifera.*
b. Inoculating the product in step a) with an entomopathogenic fungus of the species *B. bassiana* selected from any of the strains with deposit number CBS121096 (strain 193) and CBS121097 (strain 203).

In a preferred implementation of this aspect of the invention, the method also includes a sterilization step of palm by-products, which may take place before or after the fragmentation or crushing.

A third aspect of the invention relates to the use of the phytosanitary composition of the invention for the biological control of the red weevil (*Rhynchophorus ferrugineus*).

### Definitions:

*Beauveria bassiana* is a species belonging to the subdivision Deuteromycetes. The colonies of this fungus may be from white to yellow, depending on the growth media. The conidiogenous cell is globose in shape with a neck forming zig-zag. Conidia are hyaline and globose.

### BRIEF DESCRIPTION OF THE FIGURES

Figure 1: Median lethal Time (LT₅₀) of larvae of *C. dimidiatus* after coming in contact with: the product made with *B. bassiana* (F+Bb), the product without *B. bassiana* (F-Bb).
Figure 2: Cadaver of *C*. *dimidiatus* parasitised with *B. bassiana* after being in contact with the product.
Figure 3. LT₅₀ of 30-day-old *Rhynchophorus ferrugineus* larvae in contact with colonies of *B. bassiana* strains 203, 193 and 119 in plates with commercial culture medium (corn meal agar). It is noticeable that strains 203 and 193 are highly pathogenic to the weevil, unlike the 119 strain. This latter had however been found highly pathogenic to larvae of other insects such as the lepidopteran *Galleria mellonella* and the coleopteran parasitic on palm roots *C. dimidiatus.*
Figure 4. Bioassays (LT₅₀) performed with 7 to 10-day-old *Rhynchophorus ferrugineus* larvae using the two *Beauveria bassiana* products formulated (strains 203, 193). During the 10-day test, it was impossible to calculate the LT₅₀ of untreated controls.
Figure 5. Bioassays (LT₅₀) performed with 30-day-old *Rhynchophorus ferrugineus* larvae using the two *Beauveria bassiana* products formulated (strains 203, 193). During the 10-day-test, it was impossible to calculate the LT₅₀ of untreated controls.
Figure 6. Bioassays (LT₅₀) performed with *Rhynchophorus ferrugineus* adults using *Beauveria bassiana* (strains 203, 193) in plates with commercial culture medium (corn meal agar). During the 20-day-test, it was impossible to calculate the LT₅₀ of untreated controls.
Figure 7. Bioassays (LT₅₀) performed with *Rhynchophorus ferrugineus* adults using the two *Beauveria bassiana* products formulated (strains 203, 193). During the 20-day-test, it was impossible to calculate the LT₅₀ of untreated controls.
Figure 8. Effect of the storage temperature on viability (A) (% germination) and pathogenicity (B) (LT₅₀) of *B. bassiana* after 20, 60 and 180 days of storage of the product formulated.
Figure 9. Effect of the storage moisture on viability (A) (% germination) and pathogenicity (B) (LT₅₀) of *B. bassiana* after 20, 60 and 180 days of storage of the product formulated.
Figure 10. Viability of the product formulated with *B. bassiana* applied in the soil. A) Presence of *B. bassiana* (Bb) and other fungi (Others) in products formulated (F) 3 months after burial in soils either sterilised (S) or non-sterilised (NS). B) Analyses of *B. bassiana* pathogenicity (Bb) in products formulated (F), 3 months after burial in soils either sterilised (S) or non-sterilised (NS), by means of scoring the presence of *G*. *mellonella* cadavers.

### DETAILED STATEMENT OF THE INVENTION

This invention aims to develop a method to reduce the number of pest insects in the palms and related plants without causing damage to the plant or other organisms that may be beneficial for plants, in addition to increasing the survival of entomopathogenic fungi which will act as biological control agents.

Thus, the present invention is based on the use of a combination of fungi as biological control agents and by-products of agriculture, specifically date palm seed, preferably dry (of no agricultural use), so that fungi grown on the substrate can withstand adverse environmental conditions and also facilitate their field application.

The material on which the biological control agent grow is a product of palm seed, preferably *Phoenix dactylifera.* The biological control agent is *B. bassiana* of the strains 193 and 203.

The results of tests have shown that the product formulated is highly pathogenic for pests caused by the red weevil (*R. ferrugineus*) and the Coleopteran nitidulid (*C*. *dimidiatus).* Median lethal time (LT₅₀) of these species is reduced in several days compared with the fungus inoculated without plant substrate. The mortality of the red weevil (especially larvae) is much higher than that obtained in previous studies conducted by Martin-Molina *et al.* (2001) using *B. bassiana.* These authors inoculated with no substrate a commercial strain of this fungus (Naturalis-L Troy Biosciences Inc., devised for whiteflies and aphids, not coleopterans) which had not been isolated from naturally infected weevils by *B. bassiana,* unlike our case, and consequently they obtained larval mortality rates below 25%.

In a recent study by Gindin *et al.* (Gidin et al. (2006) Phytoparasitica 34(4), 370-379.), using the entomopathogens *B. bassiana* and *Metarhizium anisopliae* isolated from hosts other than *R. ferrugineus,* obtained lower mortalities than the fungal strains claimed for 7-12 day old red palm weevil larvae, by inoculations with 20 million conidia suspension per mililiter of these fungi:
- LT₅₀ (*B. bassiana* isolate Gindin *et al*.) = 5,8-6,5 days
- LT₅₀ (*M. anisopliae* isolate Gindin *et al*.) = 3,5-5 days
- LT₅₀ (*B. bassiana* from the present invention isolated from *R. ferrugineus* on *P. dactylifera* seed) = 1,75-2,5 days

### In this study the authors use a product based on rice grains, a primary product of agriculture, to sprinkle on red weevil females and check their fertility, not its pathogenicity. On the contrary, the method of this invention, which takes advantage of an abundant crop residue of the palm, has proven to be pathogenic by contact and/or by eating for larvae (young and 30 day-old) and even adults of R. ferrugineus.

In turn, the product made for this invention (the composition of the invention) comprising palm seeds has shown in the field to survive for about 2 months when applied to the apical meristem in palms with knotted leaves. Furthermore, it appears that not only is the product effective fresh, but it also withstands different storage conditions (several temperatures and moistures) for at least 2 months, and being buried in different soil samples without losing viability or pathogenicity in several cases.

### EXAMPLES

Below a series of tests performed by the inventors are shown, aimed to demonstrate the effectiveness of the invention but not to limit it.

### EXAMPLE 1. METHOD FOR PRODUCTION AND FORMULATION OF ENTOMOPATHOGENIC FUNGI ON PALM BY-PRODUCTS AND THE ANALYSIS OF ITS PATHOGENENICITY.

### 1.1 Sterilization and inoculation of agricultural by-products

To elaborate the product formulated, dried date palm seed with no perforations, which would otherwise indicate attack damage by a secondary seed pest, are used. First, the *P. dactylifera* seed are fragmented, since *B*. *bassiana* grows better in the gaps that appear inside them. Seeds were placed in a dry blender with two metal cogwheels and fragmented into small size (8x8 mm) pieces. Once the seed fragments were obtained, they were size selected by sieving and then sterilized.

Date palm seed fragments were placed in 100 ml beakers, each containing 10 grams of fragmented *P. dactylifera* seed, which were immersed in distilled water for 30 minutes to saturation. Subsequently, the excess water of the substrate was eliminated and then seed fragments were sterilized in a steam autoclave for 90 minutes at 130 ºC. The sterilised plant material was placed in sterile Petri dishes and left to stand for 2-3 hours to remove any volatile compounds that could inhibit the growth of the entomopathogenic fungus. Plates with 10 g of substrate plant were inoculated with 100 conidia (per beaker) extracted in distilled water, from a young colony (approximately 9 days old) of the entomopathogenic fungus growing in artificial growth medium CMA (corn meal agar).

Subsequently, the plates were incubated between 20 and 30ºC, preferably 25 ° C in darkness for two weeks for the fungus to grow on the substrate. Subsequently fragments of *P. dactylifera* seed inoculated with the fungus were air dried to constant weight. For this purpose Petri dishes with fresh substrate were left open in a fume hood for 24 h. Once dried substrates were stored in the same petri dishes, and the product was ready to use.

### 1.2. Production and viability of the formulates

Once agricultural by-products inoculated with the fungus had been dried, a study was performed to analyse the yield and viability of entomopathogenic fungi produced.

Two weeks after inoculation of the by-products with fungus, spores or conidia from each Petri plate were collected, by shaking for 25 minutes at 200 rpm portions of inoculated substrates in 50 ml of sterile distilled water. The resulting suspension was then filtered through sterile muslin and then by sterile glass wool to separate conidia from the substrate. The production was estimated as the number of conidia per gram of substrate, to do so number of conidia/ml in the spore suspensions was measured in a Neubauer Chamber.

To know the exact amount of conidia generated from the initial inoculum, the volume of suspension collected from each of the plates was measured with a sterile measuring cylinder, for the final estimate of the number of conidia per gram of substrate.

Furthermore, the viability of the conidia produced was estimated by germination on Petri dishes with CMA. Dilutions of the original conidia suspension to adjust the inoculum and 100 conidia were placed per plate with CMA. Three days after inoculation colonies appearing on each plate were scored.

In the following table (Table 1), production/yield and viability data of the inoculum of *B. bassiana* produced in CMA plates (commercial culture medium) and in fragments of *P. dactylifera* seed (formulated product).

**Table 1: Production (conidia.g⁻¹ substrate) and viability (percentage of conidia germination) of Beauveria bassiana produced in CMA (commercial culture medium) and in fragments of P. dactylifera seed (formulated product).**

| ***B. bassiana*** | **Production/Yield** | **Viability** |
|---|---|---|
| **CMA** | 5.54 x 10⁶ ± 2.10 x 10⁶ | 104.06 ± 43.85 |
| ***P. dactylifera* Seed** | 1.48 x 10⁸ ± 9.12 x 10⁷ | 122.9 ± 12.98 |

As shown there is an increase in production (X² = 8436, P <0.05) of B. bassiana when formulated from date palm seed culture medium with respect to commercial CMA.

### 1.3. Pathogenicity of product formulated

Pathogenicity of entomopathogenic fungi grown on palm seed were estimated in two tests performed on L5 larval stage of the lepidopteran *Galleria mellonella.*

In the first test a 1 g sample of a product formulated was shaken at 200 rpm for 25 min. with 50 ml of sterile distilled water. From this sample each *Galleria mellonella* larva was inoculated with 4x10⁴ conidia in 20 ml of distilled water applied with a micropipette.

In a second test for pathogenicity 1 g of the product formulated was added to sterile Petri dishes, to which 10 *G. mellonella* larvae were added. Subsequently, the plates were incubated at 25 ° C, scoring at two day intervals the number of dead larvae, until the mortality reached at least half of the larvae of each plate (LT₅₀). This test was performed in triplicate (3 plates).

As control for both experiments, the pathogenicity of uninoculated *P*. *dactylifera* seed (they had been additioned distilled water instead of *B*. *bassiana* conidia), was estimated on *G. mellonella* which were placed in Petri dishes. Alternatively, larvae were inoculated with water produced by the agitation (200 rpm for 25 min) of 1 g *P. dactylifera* seed in 50 ml of sterile distilled water.

In the tests, it was found that the larvae of *G*. *mellonella* have a natural mortality (LT₅₀) of about 20 days, i.e. 20 days to die 50% of the untreated population. By making contact larvae with date palm seed fragments without fungus, the LT₅₀ showed no significant differences with the untreated control larvae (Table 2). Larvae inoculated with the entomopathogenic fungus show an LT₅₀ of about 7 days, which is significantly lower than the LT₅₀ for larvae treated with water from non inoculated seed. However, larvae which have been in contact with the product formulated with the fungus show an even lower LT₅₀, which means that in a very short time half the population of larvae per plate (1 to 2 days) dies. Furthermore, by studying the bodies of these larvae it was found that 100% displayed the fungus, showing that this was the causal agent of their death. Therefore, the tests show that the product formulated is highly pathogenic for this insect.

**Table 2: Pathogenicity of B. bassiana produced in P. dactylifera seed on G. mellonella larvae. Controls (C) are also included.**

| **TREATMENT** | **LT₅₀ (DAYS)** | **% Insects sporulated** |
|---|---|---|
| **Larvae with no diet (Natural Mortality) (C)** | 20.22 ± 2.03 | - |
| **Water after shaking *P. dactylifera* Seed (C)** | 13.22 ± 6.73 | - |
| **Suspension of *B. bassiana* conidia prod. in *P. dactylifera* Seed** | 7.6 ± 0.69 | 100 |
| ***P. dactylifera* Seed (C)** | 16.83 ± 1.89 | - |
| ***P. dactylifera* Seed with *B. bassiana*** | 1.86 ± 1.50 | 100 |

### 1.4. Analysis of the pathogenicity of the product formulated with B. bassiana on palm pests in the laboratory

To show the pathogenicity of the product formulated with palm seed on a real palm pest, it was placed in contact with larvae of the coleopteran *Carpophilus dimidiatus,* which attacks the roots of young palms. Similar experiments were also performed with the red palm weevil (*Rhynchophorus ferrugineus*).

### 1.4.1. Analysis of the pathogenicity of B. bassiana against the root eating coleopteran Carpophilus dimidiatus (Reference Example).

Larvae of *C. dimidiatus* which were found inside the roots of *P. dactylifera,* where they had formed galleries were extracted and placed in Petri dishes with the product formulated (5 larvae per plate, with 3 replicates). Each plate had a different treatment:
1) Two grams of *B. bassiana* formulate (3 replicates)
2) Two grams of the formulate without fungus (3 replicates)
3) Five larvae 5 on their own/ plate (3 replicates)

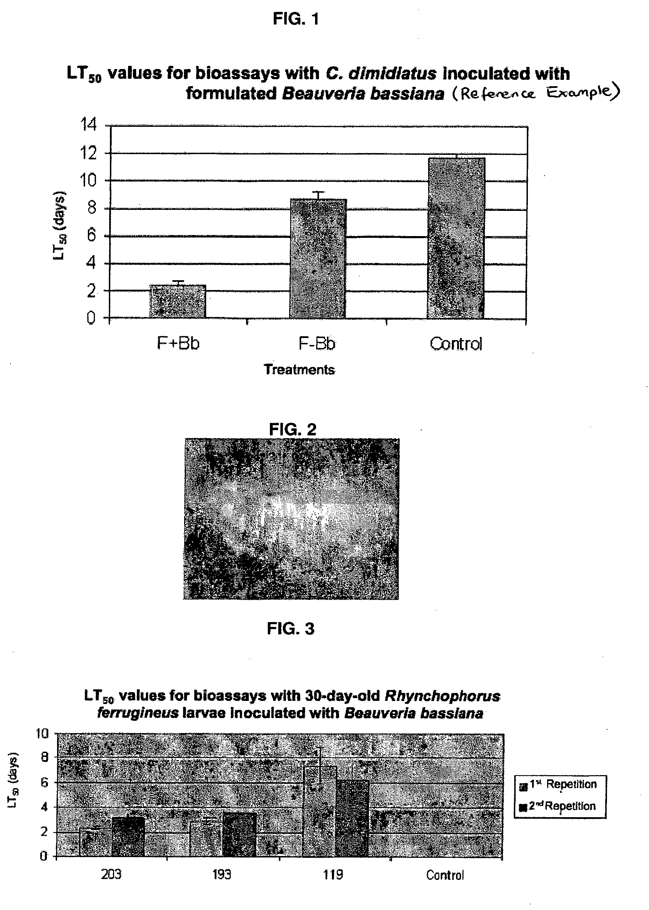

Subsequently, the day of larval death was scored (to calculate the median-lethal time LT₅₀, which is the time it takes for 50% of the insect population to die) (Fig. 1). The cadavers were surface sterilized to place them in moist chambers and checked to rule out whether the death of *C. dimidiatus* had been due to the entomopathogenic fungus or not. Afterwards the insect bodies were placed on CMA with rose bengal and antibiotics to see if the colony of the entomopathogenic fungus was present in treatments incuding *B. bassiana.*

Larvae of *C. dimidiatus* in contact with the product formulated with *B. bassiana,* died on the third day on the plate (LT₅₀ = 2.37 ± 0.41 days). After examining the bodies, 100% showed the fungus inside (Fig. 2).

Larvae in contact with the control seed, i.e. without *B. bassiana,* died after 13-14 days (LT₅₀ = 8.66 ± 0.60 days). None of the larvae displayed fungus upon incubation in moist chambers.

Controls (larvae without food in a petri dish), died after 11-12 days (LT50 = 11.17 ± 0 days). *B. bassiana* was not isolated from any of the bodies of these larvae.

Statistically significant differences between the two treatments and the control were found (H [9,2] = 7.20, p <0.05). Therefore, the product formulated with *B. bassiana* actually has a median lethal half time lower than the control, implying that is effective against these larvae which attack the young palms.

### 1.4.2. Analysis of the pathogenicity of B. bassiana against the weevil Rhynchophorus ferrugineus.

In a first stage of this analysis a large number of pathogenic strains of *B*. *bassiana* were tested as potential pathogens for *Rhynchophorus ferrugineus.* 30 day-old larvae of this weevil and CMA growth medium (a laboratory growth medium which allows optimal growth of the fungus) were used for experiments.

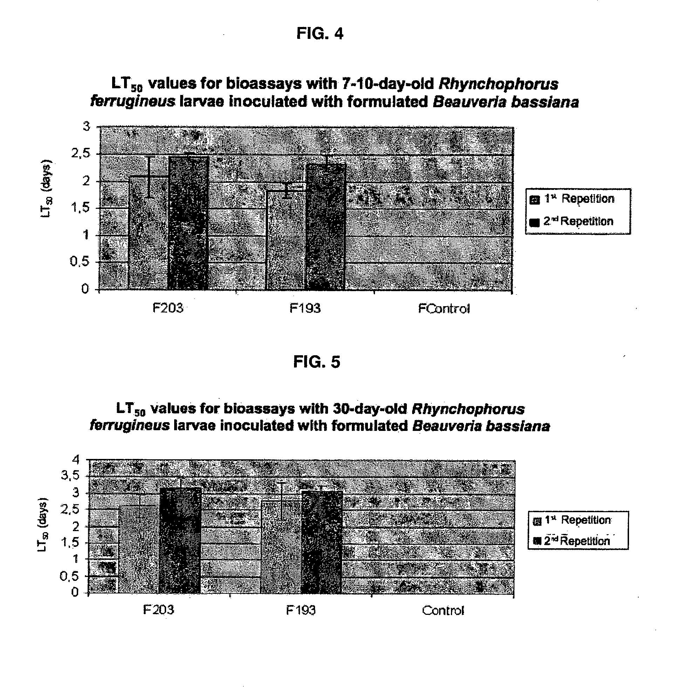

From these experiments, strains with low pathogenicity to the red weevil (e.g. 119 or strain CBS 121098, deposited in the Netherlands, "Centraalbureau voor Schimmelcultures") were discarded. Strains 193 and 203, which had proved to be the most pathogenic (Fig. 3) were selected for formulation in plant material. The high pathogenicity of these strains could be tested (Fig. 4) with the results provided by LT₅₀ pathogenicity bioassays performed on *R*. *ferrugineus* young larvae (7-10 day-old).

On the other hand, the same tests with these pathogenic strains formulated, were performed with 30 day old larvae, where the median lethal time was 3 days ca. (Fig. 5). While in the controls (larvae in contact with the seed without the fungus) larvae remained alive for at least 10 days.

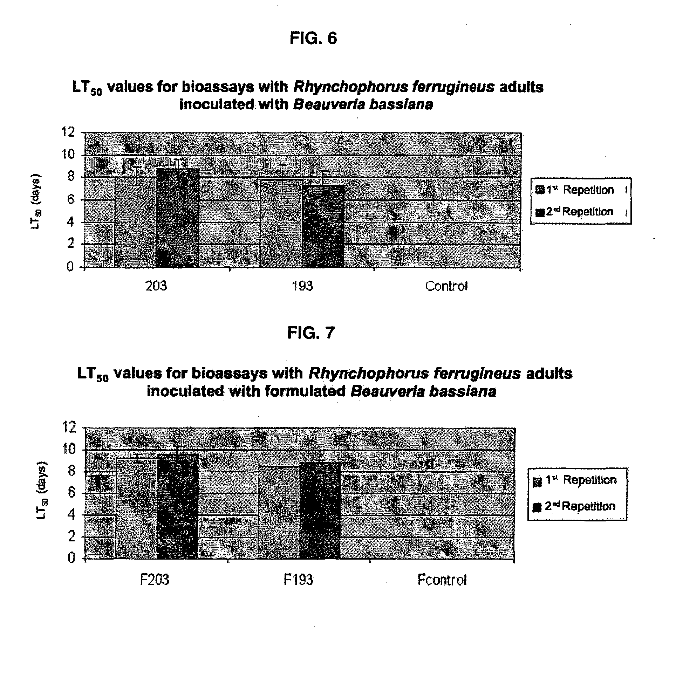

Then, a comparative study of the pathogenic strains of 203 and 193 grown on palm products (Fig. 7) and a laboratory culture medium optimal for *B*. *bassiana* such as CMA (Fig. 6), was carried out to demonstrate that the pathogenicity was maintained regardless of the substrate used. To carry out these tests red weevil adults were placed in petri dishes (9 cm in diameter), containing the product formulated with the strain to be tested (203 or 193). Eighteen adults (2 per petri dish) for 3 replicate experiments, and 18 adults for controls were used. To each petri dish 2.75 g of the product formulated with *B. bassiana* were added (the controls contained seed fragments with no fungus). The adults were placed in the plates for 30 minutes. After this time each individual was transferred separately to a moist chamber (plate with sterile filter paper moistened with 700 ml of distilled water) for 12 hours. After they were placed in individual plates with new sterile filter paper moistened with 600 µl of sterile distilled water. Since the plates were not sealed, every 2 days 300 µl of sterile distilled water were added to each plate to avoid drying of insects. It was found that there was no statistical difference in the LT₅₀ of test with the two isolates and their formulates (Table 3).

**Table 3: This table shows LT₅₀ values of laboratory tests with R. ferrugineus adults with isolates, 193 and 203 and their formulates. For statistics purposes a significance of 95% was assumed. There were no differences between different treatments**

| **Average LT₅₀ value (days)** | |
|---|---|
| | Insect stage |
| Treatment | Adults |
| 193 | 7,83 ± 1,28 |
| F193 | 8,44 ± 0,10 |
| 203 | 8,05 ± 0,81 |
| F203 | 9,22 ± 0,38 |

Furthermore, although the median lethal time for adults (between 8 and 10 days) was higher than that for the larvae (less than 3 days), it was significantly lower than in controls (product formulated without the fungus-Fcontrol - ) for insects in the same stage of development.

### EXAMPLE 2. USE AND APPLICATION OF THE PRODUCT FORMULATED IN PALM BY-PRODUCTS FOR BIOLOGICAL CONTROL OF INSECT PESTS

For the use and application of the product formulated it was found adequate a change in its development to improve its field application. This consisted on the addition of powder resulting from the crushing of the seeds. This allows the spores of entomopathogenic fungi attached to powder particles to be more easily transported by the wind to all parts of the palm where the product is being applied. Once produced the product formulated with the fungus in question, 100 g can be delivered in the heart of the palm infested by insect pests.

Once the formulated product is applied, palm can leaves can then be knotted to promote moisture and therefore the action of the fungus (at the same time this is done to obtain white palms) for a period of about 2 months. After this period seed fragments can be recovered to study if the fungus has survived this period, or if has lost pathogenicity. At the same time it is tested whether the population of insect pests has been undermined by the presence of the product.

Here's how to get the product formulated modified and its application as an example in palm trees infested by insects.

### 2.1. MODIFICATION OF THE PRODUCTION OF THE FORMULATE FOR ITS APPLICATION IN THE FIELD

As discussed above, a way of modifying the product formulated with improved spore dispersal the fungus in the field was obtained using the powder obtained after grinding dried seed. For this purpose 100g palm seed were fragmented into pieces between 1 x1 mm and 10x10 mm of size. preferably of 5x5 mm from the plastic blender (see 1.1). Twenty-five g of powder coming from the fragmentation of the seeds were also collected. The 100g portions of fragmented seed were left overnight in distilled water for saturation. The 25g seed powder portions were wrapped in foil for sterilization in the steam autoclave. Finally both the flasks with fragments and the foil envelopes containing powder were sterilized for 90 minutes at 121 ºC.

The material sterilized was left to cool for 24 hours. After this period it was re-sterilized under the same conditions and cooled again. Once cooled, sterilised powder was added to flasks with 100g of sterilised fragments and both materials were mixed. When the substrate (seed powder + seed fragments) was ready, *B. bassiana* conidia were extracted from 10 day-old colonies in CMA. Each flask was inoculated with 5 ml containing 10⁷ conidia of a given fungus. Flasks were incubated for 10 days, shaking them every 2 days to spread the fungal inoculum evenly over all the fragments. The fresh formulate obtained was ready to be applied in the palms. Alternatively an additional step prior to field application was to dry the formulate down to a moisture of 39% (Moisture = 39.47 ± 1.70%)

### 2.2. VIABILITY OF PRODUCT FORMULATED WITH B. bassiana IN STORAGE CONDITIONS AND APPLIED IN THE SOIL

### 2.1.1. Storage of the Product

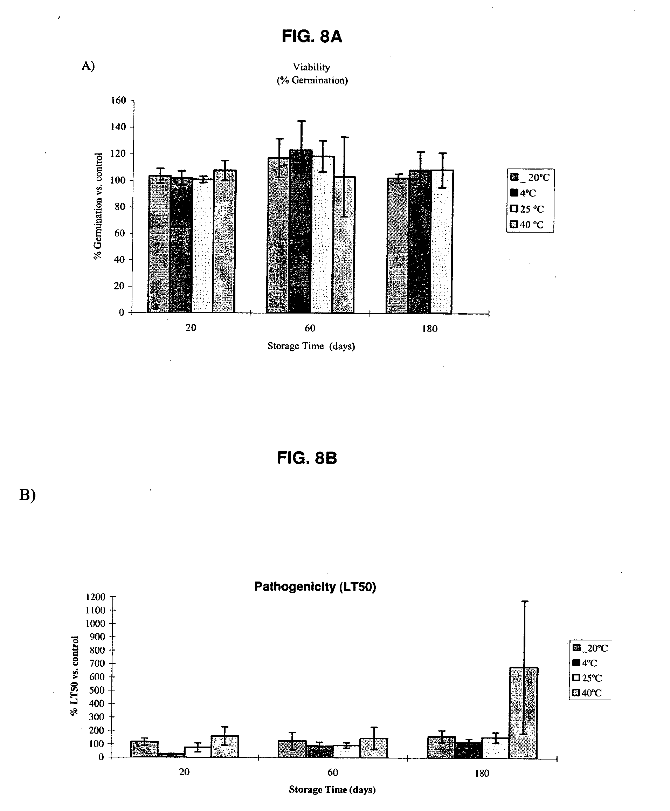

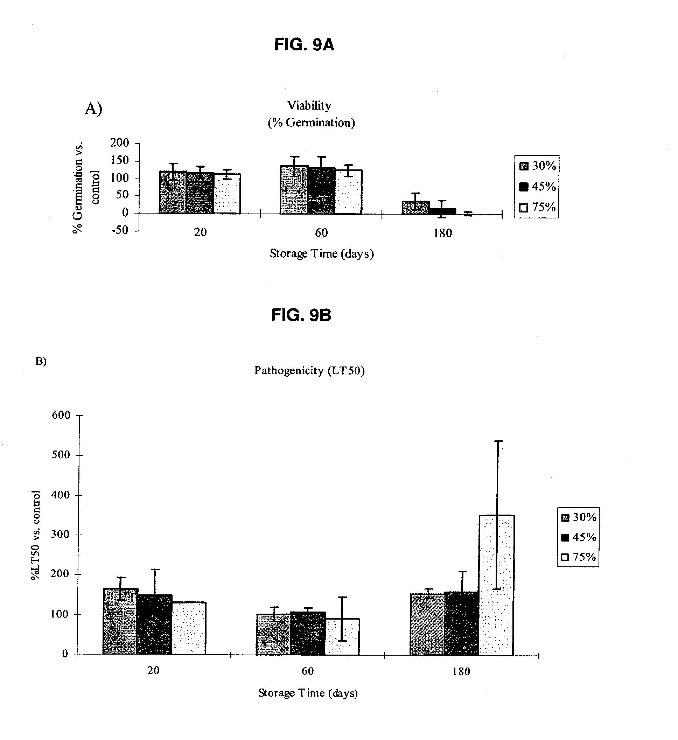

To check the viability of the product formulated under storage conditions it was analysed after 180 days storage at different temperatures and humidities. These studies showed that the date palm seeds formulated with *B. bassiana* were able to keep the fungus alive for at least 2 months at -20, 4, 25 and 40 ºC, with a humidity below 45% without losing viability or pathogenicity respect to unstored controls (Figs. 8 and 9 ).

The results showed that the pathogenicity after 20 days of storage at temperatures of -20, 4 and 25 º C of the product formulated with *B*. *bassiana* was significantly higher (X² = 9.90, P <0.05) than that of the corresponding control without storage. Similar results were obtained after 60 days of storage (X² = 9.57, P <0.05).

### 2.1.2 Application of the product formulated in the soil

The product formulated was buried for 3 months in Petri dishes under artificial (Art) or natural soil (Nat) samples, collected from different areas of the Province of Alicante. Soil samples were either left non-sterile (NS) or were sterilised (S). This experiment was to check the viability of the formulate and test the effect of biotic factors and subject it to most adverse conditions, than those arising from storage (Example 2.1.1).

After the 3 month period the seed fragments were incubated in culture medium (CMA). The viability of *B. bassiana* was then recorded as well as the presence of other fungi on the substrate. The pathogenicity (as median lethal half time) of the product (with or without *B. bassiana)* after retrieval from soil was tested using *G. mellonella* larvae.

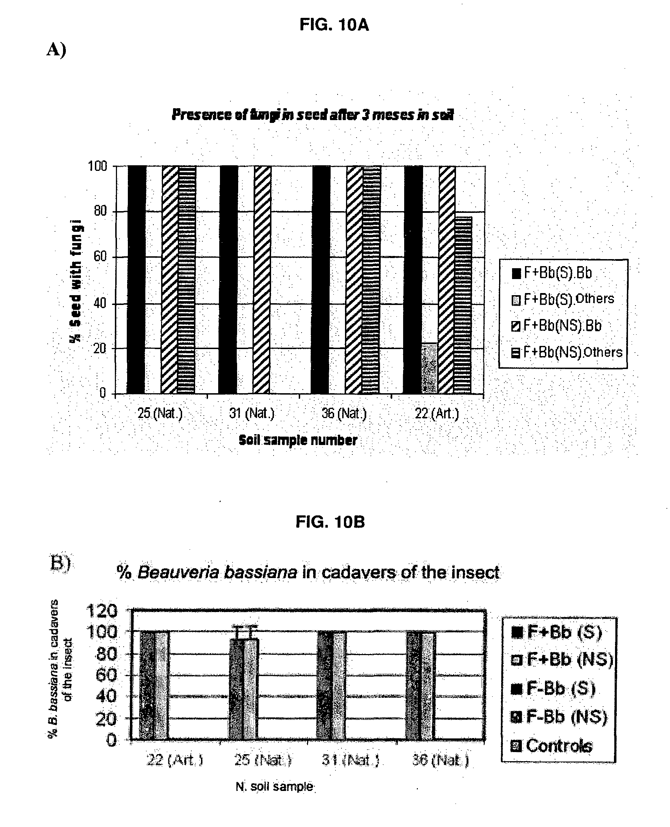

When seed fragments retrieved from soil were plated on CMA they showed viable *B. bassiana* in all cases, with few fungal contaminants, especially in sterilized soils. B. bassiana viability was 100% 3 months after burial of the formulate in both sterile and non-sterilized soil. Sometimes in seed fragments buried in non-sterilized soil samples fungi *Fusarium, Rhizopus, Alternaria* ... (Other) were recorded. However these never inhibited *B. bassiana* (Fig. 10A).

The pathogenicity of the product formulated buried in soil on *G. mellonella* larvae was similar to that of the unburied product. All larvae used in tests with the product formulated with *B. bassiana* buried in soil (either sterilized o not) displayed parasitism by the fungus (Fig. 10B)

## Claims

1. A phytosanitary composition which includes an entomopathogenic fungus of the species *Beauveria bassiana* selected from any of the strains with deposit number CBS121096 (strain 193) and CBS121097 (strain 203) and crushed or fragmented palm seed, preferably of the species *Phoenix dactylifera.*

2. The phytosanitary composition according to claim 1, which in addition includes seed powder.

3. A method for preparing the composition according to claim 1 comprising:
a. Fragmenting or crushing palm seed, preferably of the species *Phoenix dactylifera.*
b. Inoculating the product in step a) with an entomopathogenic fungus of the species *B. bassiana* selected from any of the strains with deposit number CBS121096 (strain 193) and CBS121097 (strain 203).

4. The method according to claim 3, wherein prior to inoculating the fungus to the seed fragments, powder from the crushing of the seed is added.

5. An entomopathogenic fungus selected from any of the strains with deposit number CBS121096 (strain 193) and CBS121097 (strain 203).

6. Use of the phytosanitary composition according to any of claims 1 or 2 for the biological control of *Rhynchophorus ferrugineus.*

## Patentansprüche

1. Eine phytosanitäre Zusammensetzung, die einen entomopathogenischen Pilz der Spezie *Beauveria bassiana,* ausgewählt aus einem der Stämme mit der Hinterlegungsnummer CBS121096 (Stamm 193) und CBS121097 (Stamm 203) und zerstoßenen oder fragmentierten Palmensamen, vorzugsweise von der Spezie *Phoenix dactylifera* umfasst.

2. Die phytosanitäre Zusammensetzung nach Anspruch 1, welche zusätzlich Samenpulver umfasst.

3. Verfahren zur Zubereitung der Zusammensetzung nach Anspruch 1, bestehend aus:
a. Fragmentieren oder Zerstoßen von Palmsamen, vorzugsweise von der Spezie *Phoenix dactytlifera.*
b. Inokulieren des Produkts von Schritt a) mit einem entomopathogenischen Pilz der Spezie *B*. *bassiana,* ausgewählt aus einem der Stämme mit der Hinterlegungsnummer CBS121096 (Stamm 193) und CBS121097 (Stamm 203).

4. Das Verfahren nach Anspruch 3, wobei vor dem Inokulieren des Pilzes mit den Samenfragmenten, Pulver vom Zerstoßen der Samen hinzugefügt wird.

5. Ein entomopathogenischer Pilz, ausgewählt aus einem der Stämme mit der Hinterlegungsnummer CBS121096 (Stamm 193) und CBS121097 (Stamm 203).

6. Verwendung der phytosanitären Zusammensetzung nach einem der Ansprüche 1 oder 2 für die biologische Kontrolle von *Rhynchophorus ferrugineus.*

## Revendications

1. Une composition phytosanitaire qui comprend un champignon entomopathogène de l'espèce *Beauveria bassiana* sélectionné parmi n'importe laquelle des souches déposées sous les numéros CBS121096 (souche 193) et CBS121097 (souche 203) et des graines de palmier, de préférence de l'espèce *Phoenix dactylifera,* écrasées ou fragmentées.

2. La composition phytosanitaire selon la revendication 1, qui comprend en outre de la poudre de graines.

3. Un procédé pour préparer la composition selon la revendication 1 qui consiste à :
a. Fragmenter ou écraser des graines de palmier, de préférence de l'espèce *Phoenix dactylifera.*
b. Inoculer un champignon entomopathogène de l'espèce *B*. *bassiana,* sélectionné parmi n'importe laquelle des souches déposées sous les numéros CBS121096 (souche 193) et CBS121097 (souche 203), au produit de l'étape a).

4. Le procédé selon la revendication 3, dans lequel on ajoute, avant d'inoculer le champignon aux fragments de graines, de la poudre de graines écrasées.

5. Un champignon entomopathogène sélectionné parmi n'importe laquelle des souches déposées sous les numéros CBS121096 (souche 193) et CBS121097 (souche 203).

6. Utilisation de la composition phytosanitaire selon n'importe laquelle des revendications 1 ou 2 pour le contrôle biologique du *Rhynchophorus ferrugineus.*
